# EUROPEAN PATENT SPECIFICATION

(11) **EP 1 908 822 B1**
(45) Date of publication and mention of the grant of the patent: **13.10.2010**
(21) Application number: 07114143.6
(22) Date of filing: 24.03.2004
(51) Int. Cl.: C12N 7/00, C07K 14/08

(54) **Infectious bursal disease virus mutants and vaccines**
Infektiöse Bursitis-Virus Mutanten und Impfstoffe
Virus mutant de la bursite infectieuse aviaire et des vaccins

(30) Priority: 24.03.2003 EP 03075842
(43) Date of publication of application: 09.04.2008
(62) Divisional of application: 04741440.4
(73) Proprietor: Intervet International B.V., 5831 AN Boxmeer (NL)
(72) Inventor: Mundt, Egbert, c/o BFAV, D-17498 Insel Riems (DE); Letzel, Tobias, D-16225, Eberswalde (DE); Paul, Guntram, c/o Intervet International GmbH, 50739 Köln (DE); Van Loon, Adriaan Antonius Wilhelmus Maria, 5836 BB Sambeek (NL)
(74) Representative: Claessens, John

(56) References cited:
- EP-A- 1 170 302
- WO-A-95/26196
- JACKWOOD D J ET AL: "Identification and comparison of point mutations associated in classic and variant infectious bursal disease viruses" VIRUS RESEARCH, AMSTERDAM, NL, vol. 49, no. 2, 1997, pages 131-137, XP002233748 ISSN: 0168-1702

### SEQUENCE LISTING

<110> AKZO Nobel NV
<120> An infectious bursal disease virus (IBDV) mutant expressing virus neutralising epitopes specific for classic- and variant IBDV strains
<130> 2003-002-FF
<160> 35
<170> PatentIn version 3.2 ,
<210> 1
   <211> 6
   <212> PRT
   <213> Artificial
<220>
   <223> Mutant of classic IBDV
<400> 1
   Gly Gly Gln Ala Gly Asp
   1               5
<210> 2
   <211> 6
   <212> PRT
   <213> Artificial
<220> <223> Mutant of classic IBDV
<400> 2
   Gly Gly Gln Ala Gly Glu
   1 5
<210> 3
   <211> 6
   <212> PRT
   <213> Artificial
<220>
   <223> Mutant of classic IBDV
<400> 3
   Asp Gly Gln Ala Gly Asp
   1 5
<210> 4
   <211> 6
   <212> PRT
   <213> Artificial
<220>
   <223> Mutant of classic IBDV
<400> 4 <210> 5
   <211> 6
   <212> PRT
   <213> Artificial
<220>
   <223> Mutant of classic IBDV
<400> 5
<210> 6
   <211> 6
   <212> PRT
   <213> Artificial
<220>
   <223> Mutant of classic IBDV
<400> 6
   Gly Gly Gln Glu Gly Asp
   1 5
<210> 7
   <211> 6
   <212> PRT
   <213> Artificial
<220>
   <223> Mutant of classic IBDV
<400> 7
   Asp Gly Gln Glu Gly Asp
   1 5
<210> 8
   <211> 6
   <212> PRT
   <213> Artificial
<220>
   <223> Mutant of classic IBDV
<400> 8
   Asn Gly Gln Glu Gly Asp
   1 5
<210> 9
   <211> 6
   <212> PRT
   <213> Artificial
<220>
   <223> Mutant of classic IBDV
<400> 9
   Asn Gly Gln Glu Gly Glu
   1 5
<210> 10
   <211> 6
   <212> PRT
   <213> Artificial
<220>
   <223> Mutant of classic IBDV
<400> 10
   Gly Gly Gln Ala Gly Asp
   1               5
<210> 11
   <211> 6
   <212> PRT
   <213> Artificial
<220>
   <223> Mutant of classic IBDV
<400> 11
   Gly Gly Gln Ala Gly Glu
   1               5
<210> 12
   <211> 6
   <212> PRT
   <213> Artificial
<220>
   <223> Mutant of classic IBDV
<400> 12
   Asp Gly Gln Ala Gly Asp
   1               5
<210> 13
   <211> 6
   <212> PRT
   <213> Artificial
<220>
   <223> mutant of pD78A
<400> 13
   Asp Gly Gln Ala Gly Glu
   1               5
<210> 14
   <211> 6
   <212> PRT
   <213> Artificial
<220>
   <223> Mutant of classic IBDV
<400> 14
   Asp Gly Gln Glu Gly Asp
   1               5
<210> 15
   <211> 6
   <212> PRT
   <213> Artificial
<220>
   <223> Mutant of classic IBDV
<400> 15
   Asn Gly Gln Ala Gly Asp
   1               5
<210> 16
   <211> 6
   <212> PRT
   <213> Artificial
<220>
   <223> Mutant of classic IBDV
<400> 16
   Asn Gly Gln Ala Gly Glu
   1               5
<210> 17
   <211> 6
   <212> PRT
   <213> Artificial
<220>
   <223> Mutant of classic IBDV
<400> 17
   Asn Gly Gln Glu Gly Asp
   1               5
<210> 18
   <211> 6
<212> PRT
   <213> Artificial
<220>
   <223> Mutant of classic IBDV
<400> 18
   Asn Gly Gln Glu Gly Glu
   1               5
<210> 19
   <211> 31
   <212> PRT
   <213> Infectious bursal disease virus
<400> 19
   Ser Asp Arg Pro Arg Val Tyr Thr Ile Thr Ala Ala Asp Asp Tyr Gln
   1               5                   10                  15
   Phe Ser Ser Gln Tyr Gln Pro Gly Gly Val Thr Ile Thr Leu Phe
               20                  25                  30
<210> 20
   <211> 45
   <212> DNA
   <213> Artificial
<220>
   <223> oligonucleotide for site-directed mutagenesis
<400> 20
   gaccatgaca tctgatcccc tgcctgaccg tcacttttgg aggtc 45
<210> 21
   <211> 45
   <212> DNA
   <213> Artificial
<220>
   <223> oligonucleotide for site-directed mutagenesis
<400> 21
   gaccatgaca tctgttcccc tgcctgaccg tcacttttgg aggtc 45
<210> 22
   <211> 45
   <212> DNA
   <213> Artificial
<220>
   <223> oligonucleotide for site-directed mutagenesis
<400> 22
   gaccatgaca tctgatcccc ttcctgaccg tcacttttgg aggtc 45
<210> 23
   <211> 45
   <212> DNA
   <213> Artificial
<220>
   <223> oligonucleotide for site-directed mutagenesis
<400> 23
   gaccatgaca tctgttcccc ttcctgaccg tcacttttgg aggtc 45
<210> 24
   <211> 45
   <212> DNA
   <213> Artificial
<220>
   <223> oligonucleotide for site-directed mutagenesis
<400> 24
   gaccatgaca tctgttcccc tgcctgacca ccacttttgg aggtc 45
<210> 25
   <211> 45
   <212> DNA
   <213> Artificial
<220>
   <223> oligonucleotide for site-directed mutagenesis
<400> 25
   gaccatgaca tctgatcccc ttcctgacca ccacttttgg aggtc 45
<210> 26
   <211> 45
   <212> DNA
   <213> Artificial
<220>
   <223> oligonucleotide for site-directed mutagenesis
<400> 26
   gaccatgaca tctgttcccc ttcctgacca ccacttttgg aggtc 45
<210> 27
   <211> 45
   <212> DNA
   <213> Artificial
<220>
   <223> oligonucleotide for site-directed mutagenesis
<400> 27
   gaccatgaca tctgatcccc tgcctgaccg ttacttttgg aggtc 45
<210> 28
   <211> 45
   <212> DNA
   <213> Artificial
<220>
   <223> oligonucleotide for site-directed mutagenesis
<400> 28
   gaccatgaca tctgttcccc tgcctgaccg ttacttttgg aggtc 45
<210> 29
   <211> 45
   <212> DNA
   <213> Artificial
<220>
   <223> oligonucleotide for site-directed mutagenesis
<400> 29
   gaccatgaca tctgatcccc ttcctgaccg ttacttttgg aggtc 45
<210> 30
   <211> 45
   <212> DNA
   <213> Artificial
<220>
   <223> oligonucleotide for site-directed mutagenesis
<400> 30
   gaccatgaca tctgttcccc ttcctgaccg ttacttttgg aggtc 45
<210> 31
   <211> 39
   <212> DNA
   <213> Artificial
<220>
   <223> oligonucleotide for site-directed mutagenesis
<400> 31
   attgttaccc caccggtttg gtactgtgat gagaattgg 39
<210> 32
   <211> 30
   <212> DNA
   <213> Artificial
<220>
   <223> oligonucleotide for site-directed mutagenesis
<400> 32
   gattgttacc ccaccgcttt ggtactgtga 30
<210> 33
   <211> 36
   <212> DNA
   <213> Artificial
<220>
   <223> oligonucleotide for site-directed mutagenesis
<400> 33
   gtcactgcta ggctcccaga tgccgaccat gacatc 36
<210> 34
   <211> 6
   <212> PRT
   <213> Artificial
<220>
   <223> Mutant of classic IBDV
<400> 34
   Asp Gly Gln Glu Gly Glu
   1               5
<210> 35
   <211> 6
   <212> PRT
   <213> Artificial
<220>
   <223> Mutant of classic IBDV
<400> 35
   Gly Gly Gln Glu Gly Glu
   1               5

## Description

The present invention is concerned with a classic infectious bursal disease virus (IBDV) mutant and a vaccine comprising such a classic IBDV mutant.

Infectious bursal disease virus (IBDV) is a member of the Birnaviridae family. Viruses in this family have a very similar genomic organisation and a similar replication cycle. The genomes of these viruses consist of two segments (A and B) of double-stranded (ds) RNA. The larger segment A encodes a polyprotein, which is cleaved by autoproteolysis to form mature viral proteins VP2, VP3 and VP4. VP2 and VP3 are the major structural proteins of the virion. VP2 is the major host-protective immunogen of birnaviruses, and contains the immunogenic regions responsible for the induction of virus neutralising antibodies.

For IBDV, two serotypes exist, serotype 1 and 2. The two serotypes can be differentiated by virus neutralisation (VN) tests. Serotype 1 viruses have been shown to be pathogenic to chickens, while serotype 2 IBDV only causes sub-acute disease in turkeys. Infectious Bursal disease (IBD), also called Gumboro disease, is an acute, highly-contagious viral infection in chickens that has lymphoid tissue as its primary target with a selective tropism for cells of the bursa of Fabricius. The morbidity rate in susceptible flocks is high, with rapid weight loss and moderate to high mortality rates. Chicks that recover from the disease may have immune deficiencies because of the destruction of the bursa of Fabricius, which is essential to the defence mechanism of the chicken. The IBD-virus causes severe immunosuppression in chickens younger than 3 weeks of age and induces bursal lesions in chicks up to 3 months old.

For many years the disease could be prevented by inducing high levels of antibodies in breeder flocks by the application of an inactivated vaccine, to chickens that had been primed with attenuated live IBDV vaccine. This has kept economic losses caused by IBD to a minimum. Maternal antibodies in chickens derived from vaccinated breeders prevent early infection with IBDV and diminish problems associated with immunosuppression. In addition, attenuated live vaccines have also been used successfully in commercial chicken flocks after maternal antibodies had declined.

Historically, IBD viruses consisted of only one type that is known as "classic" IBD virus. However, in the mid-1980s acute disease in flocks vaccinated with vaccines based on classic IBDV was observed, in particular in the US. It was found that this disease was caused by IBD viruses that had a different immunogenic make-up. These new viruses probably emerged as a result of genetic drift. The emergence of these so-called "variant" IBDV strains required the design of new IBD vaccination programmes, because the classic IBDV vaccine strains could not induce an adequate cross-protection. The most important variant subtypes of serotype 1 IBDVs identified in the past were the Delaware-E, GLS, RS/593 and DS326 variants. The variant strains can be identified and distinguished from classic strains by a virus neutralisation test, a panel of monoclonal antibodies or RT-PCR.

Delaware variant-E was reported by Rosenberger et al. (Proc. 20th Natl. Meet. on Poultry Health and Condemnations; Ocean City, MD, USA, 94-101, 1985) and Snyder et al. (Avian Diseases 32, 535-539, 1985). GLS virus was isolated in the USA in 1987 and DS326 (GLS-like) was isolated in the USA in 1988 (Snyder et al., Arch. Virol. 127, 89-101, 1992 and van Loon et al. Proceedings of the International symposium on infectious bursal disease and chicken infectious anaemia, Rauischholzhausen, Germany, 179-187, 1994). Strain RS/593 (variant-E like) was also isolated in the USA, in 1993 (Snyder, et al. Proceedings of the International symposium on infectious bursal disease and chicken infectious anaemia, Rauischholzhausen, Germany, 65-70, 1994).

A panel of virus neutralising monoclonal antibodies (moab) is commonly used in the art in an antigen-capture enzyme immuno assay (AC-ELISA) to identify the various IBDV types. The reactivity pattern of these moabs with the existing IBDV strains is summarised in Table 1 below.

**Table 1: The different variant IBDV strains as determined by the moab panel pattern**

| Strain↓Moab → | 8 | B69 | R63 | 10 | BK9 | 67 | 57 | 44A1 | 179 |
|---|---|---|---|---|---|---|---|---|---|
| | | | | | | | | | |
| Classic | + | + | + | + | - | - | - | + | + |
| Delaware | + | - | + | - | + | + | - | + | + |
| variant (-E) | | | | | | | | | |
| RS/593 | + | - | - | - | - | + | - | - | + |
| GLS | + | - | - | + | - | - | + | + | + |
| DS326 | + | - | - | + | - | - | + | + | - |

VN moabs R63 and B69 neutralise classic IBDV strains to high titres and moab B69 specifically binds to classic strains. moab BK9 uniquely binds to Delaware variant-E strains. A positive reaction by moab 57 can be used to separate the GLS- and DS326 strains from classic- and Delaware variant strains. These and other moabs are generally used in the field to distinguish between IBDV (variant) strains by determining the reaction pattern of the panel of available moabs. The hybridomas secreting the moabs are also available from the ATCC (Rockville, USA) under the following accession numbers: R63 (HB-9490), 8 (HB-10174), B29 (HB-9746), BK-9 (HB-10157), 67 (HB-11122), 57 (HB-10156), B69 (HB-9437) and 179 (HB-10158). Variant IBDV strains and hybridomas are also available from the Collection Nationale de Cultures de Microorganismes of the Institute Pasteur, Paris, France under the following accession no.'s: DS 326 (I-910), GLS (I-792 and I-793) and moab 10 (I-2812).

Although the importance of some regions and distinct amino acids within the VP2 protein of IBDV for the antigenic variation between IBDV strains has been proposed (Vakharia et al., Virus Research 31, 265-273, 1994 and Snyder et al., Avian Diseases 38, 701-707, 1994), the mandatory presence of all the amino acids for the formation of IBDV neutralising epitopes has not been determined yet. In Virus Research 49, 131-137, 1997, Vakharia et al. reported that amino acid proline is present in variant E IBDV. In international patent application WO 95/26196 Vakharia et al. suggested the involvement of amino acids 286 (lle), 318( Asp) and 323 (Glu) in the context of the binding of a variant E VP2 protein with moab 67. In addition, multiple amino acid sequence differences between VP2 proteins of classic- and variant VP2 IBDV are disclosed. Amino acid positions 222 (Pro), 249 (Gln) and 254 (Gly) were considered relevant for the formation of the B69 epitope in a GLS VP2 protein.

EP 1170302 (Akzo Nobel N.V.) discloses the preparation of a variant E IBDV mutant with improved immunogenicty against classic IBDV strains. This IBDV mutant was obtained by introducing mutations in the variant E VP2 coding region in the codons for the amino acids 253 (Gln to His), 284 (Ala to Thr) and 254 (Ser to Gly), 270 (Ala to Thr). None of these variant E IBDV mutants express a VP2 protein that binds with moab B69.

However, no information is disclosed therein that allows the generation of a classic IBDV mutant that also expresses a variant E virus neutralising epitope. Such a mutant would be very useful in vaccines to induce protection against disease caused by both classic- and variant IBDV strains in the field.

In the present invention a new IBDV mutant has been constructed based on a classic IBDV by introducing mutations in the VP2 coding region such that the VP2 protein expressed by the virus comprises virus neutralising epitope 67 that is typical for variant IBDVs. The inventors found that in the context of a classic VP2 protein the reactivity with moab 67 is influenced by amino acid sequences located about 100 amino acids apart. Furthermore, the assumption made by Vakharia et al. that 286 (Ile), 318( Asp) and 323 (Glu) influence the presence of the 67 epitope is not correct. In fact the inventors have found that the emergence of the 67 epitope in the context of a classic IBDV VP2 protein depends on the exchange of proline at position 222 to serine or threonine and on the presence of certain amino acid sequences in positions 318-322. The exchange of proline to serine or threonine at position 222 in the presence of the amino acid sequences naturally present in a classic IBDV VP2 protein at positions 318-323 (Gly-Gly-Gln-Ala-Gly-Asp) lead to the emergence of the 67 epitope in a classic IBDV VP2 protein.

Therefore, the present invention provides a classic infectious bursal disease virus mutant that expresses a VP2 protein that binds with monoclonal antibody (moab) B69, **characterised in that** the VP2 protein also binds with moab 67, secreted by hybridoma cell lines HB-9437 and HB-11122, deposited at the ATCC, Rockville, USA, respectively.

The IBDV VP2 protein consists of 512 amino acids and is located on the polyprotein at amino acid positions 1-512. The nucleotide sequences of (the complete segment A comprising) the VP2 coding region and the corresponding amino acid sequences of the VP2 protein of many classic IBDV strains have been determined (US 5,871,744, EP 887,412 for D78; NCBI GeneBank)

All known moabs showing virus neutralising properties, including moab B69 that is specific for classic IBDV strains, are exclusively raised against the VP2 protein and recognise conformational-dependent epitopes. Comparison of many classic- and variant VP2 amino acid sequences, in particular of variant E and GLS, are disclosed in Vakharia et al. (1994, supra) and Heine et al. (J. Gen. Virol. 72, 1835-1843, 1991). Although biological variation among virus strains exist, within the classic type of IBDV strains the amino acid sequence of the VP2 protein is strongly conserved. Amino acid homology between classic VP2 amino acid sequences varies between 98%-99.4% over the full length of the protein (based on a comparison with the VP2 amino acid sequence of IBDV strain D78). A central region within the VP2 protein was identified which contains the most variable part of VP2. This region is located at amino acid positions 206 to 350 (Bayliss et al., J. Gen. Virol. 71, 1303-1312, 1987) and amino acid homology between classic VP2 amino acid sequences within this region varies between 95.9%-97.9% (based on a comparison with the VP2 amino acid sequence of IBDV strain D78). Within this central region two hypervariable regions are identified, at positions 212-224 and 314-326, in which most of the amino acid changes between different types of IBDV strains occur (Vakharia et al., 1994, supra). Despite this, most classic IBDV strains comprise an amino acid sequence in the region 200-230 that is identical to that in VP2 of IBDV strain D78: Ser-Asp-Arg-Pro-Arg-Val-Tyr-Thr-Ile-Thr-Ala-Ala-Asp-Asp-Tyr-Gln-Phe-Ser-Ser-Gln-Tyr-Gln-Pro-Gly-Gly-Val-Thr-Ile-Thr-Leu-Phe (SEQ ID no. 19). Sequences are analyzed herein with the Wisconsin Package, version 8 (Genetics Computer Group, Madison, Wis.)

Therefore, a classic IBDV is defined herein as an isolated IBDV comprising a VP2 coding region that expresses a VP2 protein that is able to bind with moab B69.

More in particular, a classic IBDV is defined as an isolated IBDV that comprises a VP2 amino acid sequence at positions 200-230 that is the same as that of strain D78 (SEQ ID no. 19).

The reaction of a moab with an IBDV can be determined by means of an AC-ELISA that is commonly used in the art for this purpose, such as described by Snyder et al. (1992, supra) and van der Marel et al. (Dtsch. Tierartzl. Wschr. 97, 81-83, 1990).

Alternatively, the reaction of an IBDV with a moab can also be determined by means of an immunofluoresence assay as described in the Example 1.

A preferred classical IBDV mutant according to the invention expresses a VP2 protein that in addition to moab B69 and moab 67 binds with moab R63, secreted by hybridoma cell line HB-9490, deposited at the ATCC, Rockville, USA. The moab R63 is able to neutralise both classic- and variant E strains of IBDV.

The present invention for the first time identifies which amino acid residues in the context of a classic VP2 protein are required and sufficient for (i) the formation of a neutralising epitope that binds with moab R63 (classic and variant E), (ii) the additional formation of a neutralising epitope that binds with moab 67 (variant E) and (iii) for the additional formation of a neutralising epitope that binds with moab 57 (GLS).

The inventors prepared a number of the IBDV mutants as defined above by introducing mutations in the VP2 coding region of a classic IBDV strain (Example 1). The results obtained demonstrate that identical epitopes can be folded by different amino acid sequences, whereas other amino acid sequences fail to generate the variant epitopes. Therefore, a specific coding capacity exists for a specific epitope. A summary of the relevant amino acid sequences that are required for the appropriate folding of the 67, 57 and R63 epitopes is presented in Table 2 (SEQ ID No.'s 1-5, 6-9 and 10-18, respectively). The information provided in Table 2 allows the skilled person to generate classic IBDV mutants capable of expressing a VP2 protein that in addition to virus neutralising epitope B69 also comprises virus neutralising epitope 67 that is specific for variant E IBDV strains.

**Table 2: Summary of amino acids essential for epitope folding**

| Epitope | aa 222 | aa 318 | aa 319 | aa 320 | aa 321 | aa 322 | aa 323 | aa 330 | SEQ ID no. (318-323) |
|---|---|---|---|---|---|---|---|---|---|
| 67 | S or T | G | G | Q | A | G | D | R or S | 1 |
| 67 | S or T | G | G | Q | A | G | E | R or S | 2 |
| 67 | S or T | D | G | Q | A | G | D | R or S | 3 |
| 67 | S or T | D | G | Q | A | G | E | R or S | 4 |
| 67 | S or T | N | G | Q | A | G | E | R or S | 5 |
| | | | | | | | | | |
| 57 | P, S or T | G | G | Q | E | G | D | R or S | 6 |
| 57 | P, S or T | D | G | Q | E | G | D | R | 7 |
| 57 | P, S or T | N | G | Q | E | G | D | R | 8 |
| 57 | P, S or T | N | G | Q | E | G | E | R | 9 |
| | | | | | | | | | |
| R63 | P, S or T | G | G | Q | A | G | D | R or S | 10 |
| R63 | P, S or T | G | G | Q | A | G | E | R or S | 11 |
| R63 | P, S or T | D | G | Q | A | G | D | R or S | 12 |
| R63 | P, S or T | D | G | Q | A | G | E | R or S | 13 |
| R63 | P, S or T | D | G | Q | E | G | D | S | 14 |
| R63 | P, S or T | N | G | Q | A | G | D | R or S | 15 |
| R63 | P, S or T | N | G | Q | A | G | E | R or S | 16 |
| R63 | P, S or T | N | G | Q | E | G | D | S | 17 |
| R63 | P, S or T | N | G | Q | E | G | E | S | 18 |

Therefore, a classic IBDV mutant of the invention is a mutant that binds with moab B69 and moab 67, and comprises one or more mutations in a classical VP2 coding region, such that the coding region comprises,
(i) a codon for the amino acid at position 222 encoding serine or threonine, and
(ii) a nucleotide sequence encoding an amino acid sequence shown in any of the SEQ ID. no. 2-5 at positions 318-323.

It was found that the amino acid sequence at positions 318-323 as shown in Table 2 resulted in a proper folding of the 67 epitope only in case the amino acid at position 222 (proline) was changed to serine or threonine, implying that proper folding of the epitope is influenced by amino acids located 100 positions apart.

Although the amino acid at position 330 is not critical, an advantageous IBDV mutant further comprises a codon encoding the amino acid arginine or serine at this position.

A further advantageous property of a classic IBDV mutant as defined above is that such a mutant also expresses a VP2 protein having the required amino acid sequence for the proper folding of the R63 virus neutralising epitope. It is demonstrated in the Table 2 and Example 1 that an IBDV that expresses a VP2 protein having an amino acid sequence at positions 318-323 as shown in any of the SEQ ID no. 14-15 and 17-18 (in addition to an serine or threonine at position 222) displays epitope R63 but fails to display epitope 67.

A further surprising observation made by the inventors is that an exchange of an amino acid in the VP2 coding region at positions 318-323 of a classic IBDV strain results in a decrease of the growth properties of such mutants. Such mutants display an attenuated phenotype for chickens and can advantageously be used as vaccine candidates with improved safety properties, in particular in vaccines that are administered via the in ovo route. Therefore, the present invention also provides a classic IBDV mutant that comprises one or more mutations in a classic VP2 coding region, such that the coding region comprises a nucleotide sequence encoding an amino acid sequence at positions 318-323 that is different from the natural amino acid sequence Gly-Gly-Gln-Ala-Gly-Asp (SEQ ID no. 1). Preferably, these classic IBDV mutants comprise an amino acid sequence at these positions as shown in any of the SEQ ID No. 2-5, optionally, with an amino acid at position 222 and 330 as defined above.

A classic IBDV mutant according to the invention can be prepared by introducing the required mutations in a VP2 coding region derived from any classic IBDV strain isolatable from the field or used in vaccines. Suitable IBDV strains include the well-known IBDV strains present in commercially available vaccines, such as D78, PBG98, 228E and 89-03 (Intervet Interntional B.V.). IBDV strain D78 (US patent no. 4,530,831) is also available from the ATCC under accession no. VR-2041. The nucleotide sequence of the complete segment A of strain D78, including the VP2 coding region, and the amino acid sequence of the corresponding (poly)protein is disclosed in US patent no. 5,871,744 and EP application 887,412.

In particular, a classic IBDV mutant is provided that comprises one or more mutations in a VP2 coding region of IBDV strain D78.

A further preferred classic IBDV mutant according to the present invention comprises the complete genetic backbone of the segment A of a classic IBDV strain, including the mutated classic VP2 coding region as described above. More in particular, a classic IBDV mutant as defined above is derived from IBDV strain D78.

However, a classic IBDV mutant according to the invention can also be based on the genetic backbone of a variant IBDV strain, such as a variant E or GLS strain. In such a "chimeric" classic IBDV mutant, the VP2 coding sequences on the genetic backbone of segment A of a variant IBDV strain are replaced by the corresponding, relevant classic VP2 coding sequences that additionally comprise the desired mutations that are responsible for the new variant epitopes on the classic IBDV mutant.

The generation of a classic IBDV mutant according to the invention can be achieved by means of the recently established infectious cRNA system for IBDV (Mundt and Vakharia, Proc. Natl. Acad. Sci. USA 93, 11131-11136, 1996). This reverse genetics system provides the possibility to introduce mutations in the RNA genome of the IBDV. The most important step in this reverse genetics system is to provide full length cDNA clones of the segments A and B of the IBDV, including the nucleotides of the 5'- and 3'- ends of both these segments. After cloning procedures, the full length sequences of segment A and B are operably linked to a promoter which is able to bind a DNA dependent RNA polymerase, such as the T7, SP6 or T3 polymerase, the T7 promoter being preferred. The DNA dependent polymerase is able to describe viral cRNA from full length cDNA clones of segment A and B, respectively. This cRNA is able to induce replication of the virus and the isolation of viable virus. This procedure can be performed with every natural occurring IBDV.

Reverse genetics systems have been described for various IBDV strains, such as D78 (Yao et al., J. Virol. 72, 2647-2657, 1998), strain HK46 (Lim et al., J. Virol. 73, 2854-2862, 1999), CEF 94 (Boot et al., Virology 265, 330-341, 1999) and UK661 (van Loon et al., J. Gen. Virol. 83, 121-129, 2002).

The desired mutations can be introduced into the IBDV genome by means of methods generally known in the art for this purpose. In particular, the mutation(s) are introduced by means of site-directed mutagenises. Methods for introducing a mutation in the IBDV genome are described herein, but are also generally used in the art (Mundt and Vakharia, 1996, supra; Yao et al., J. Virology 72, 2647-2654, 1998; Mundt et al., 1999, supra; EP patent application no. 1170302; Current Protocols in Molecular Biology, eds.: F. M. Ausubel et al., Wiley N.Y., 1995 edition, pages 8.5.1.-8.5.9.and Kunkel et al. in Methods in Enzymology vol. 154, 376-382, 1987).

The numbers used herein to indicate the amino acid positions refer to numbering of the amino acids in the IBDV polyprotein as commonly used in the art. The numbers indicating the nucleotide positions are based on the complete nucleotide sequence of the segment A of the IBDV genome as described by Mundt and Müller (J. Gen. Virol. 77, 437-443, 1995; NCBI accession number X 84034).

The segment B of a classic IBDV mutant according to the invention can be derived from any IBDV strain, preferably from a classic IBDV strain, most preferably from strain D78 or P2 (US patent 5,871,744 and EP patent application no. 887412).

As demonstrated in the Examples, the classic IBDV mutant according to the invention displays an immunogenic make-up that is not observed before for classic IBDV strains. The new classic IBDV mutant may form the basis of a new type of IBDV vaccine that can effectively protect poultry against disease conditions resulting from the infection by both classic- and variant IBDV strains. Therefore, another aspect of this invention is a vaccine for use in the protection of poultry against disease caused by IBDV infection, **characterised in that** the vaccine comprises a classical IBDV mutant as defined above, together with a pharmaceutical acceptable carrier or diluent.

The classical IBDV mutant can be incorporated into the vaccine as live attenuated or inactivated virus.

A vaccine according to the invention can be prepared by conventional methods such as for example commonly used for the commercially available live- and inactivated IBDV vaccines. Briefly, a susceptible substrate is inoculated with a classical IBDV mutant according to the invention and propagated until the virus replicated to a desired infectious titre after which IBDV containing material is harvested, optionally inactivated, and mixed with a pharmaceutical acceptable carrier or diluent.

Every substrate which is able to support the replication of IBDVs can be used to prepare a vaccine according to the present invention, including primary (avian) cell cultures, such as chicken embryo fibroblast cells (CEF) or chicken embryo liver cells (CEL), mammalian cell lines such as the VERO cell line or the BGM-70 cell line, or avian cell lines such as QT-35, QM-7 or LMH. Usually, after inoculation of the cells, the virus is propagated for 3-14 days, after which the cell culture supernatant is harvested, and if desired filtered or centrifuged in order to remove cell debris.

The classical IBDV mutant can also be propagated in embryonated chicken eggs.

If desired, attenuation of the classical IBDV can be obtained by standard serial passaging of the virus in cell cultures, for example in the primary cell cultures or established cell lines mentioned above (Bayyari et al., Avian Diseases 40, 516-532, 1996; Tsai et al., Avian diseases 36, 415-422, 1992).

Alternatively, the classical IBDV can be propagated in vivo in infected chickens followed by the isolation of the bursa of Fabricius from these infected animals, mixing it with diluent and homogenizing the mixture. IBDV propagated in this way commonly forms the basis of an inactivated vaccine.

The vaccine according to the invention containing the live virus can be prepared and marketed in the form of a suspension or in a lyophilised form and additionally contains a pharmaceutically acceptable carrier or diluent customary used for such compositions. Carriers include stabilisers, preservatives and buffers. Suitable stabilisers are, for example SPGA, carbohydrates (such as sorbitol, mannitol, starch, sucrose, dextran, glutamate or glucose), proteins (such as dried milk serum, albumin or casein) or degradation products thereof. Suitable buffers are for example alkali metal phosphates. Suitable preservatives are thimerosal, merthiolate and gentamicin. Diluents include water, aqueous buffer (such as buffered saline), alcohols and polyols (such as glycerol).

If desired, the live vaccines according to the invention may contain an adjuvant. Examples of suitable compounds and compositions with adjuvant activity are the same as mentioned below.

Although administration by injection, e.g. intramuscularly, subcutaneously or in ovo of the live vaccine according to the present invention is possible, the vaccine is preferably administered by an inexpensive mass application route commonly used for IBDV vaccination. For IBDV vaccination this route includes drinking water, spray and aerosol vaccination.

Alternatively, the present invention provides a vaccine comprising the variant IBDV in an inactivated (killed) form. An advantage of an inactivated IBDV vaccine is the high levels of protective antibodies of long duration that can be obtained.

The aim of inactivation of the viruses harvested after the propagation step is to eliminate reproduction of the viruses. In general, this can be achieved by chemical or physical means well known in the art.

A vaccine containing the inactivated variant IBDV can, for example, comprise one or more of the above-mentioned pharmaceutically acceptable carriers or diluents suited for this purpose.

Preferably, an inactivated vaccine according to the invention comprises one or more compounds with adjuvant activity. Suitable compounds or compositions for this purpose include aluminium hydroxide, -phosphate or -oxide, oil-in-water or water-in-oil emulsion based on, for example a mineral oil, such as Bayol F® or Marcol 52® or a vegetable oil such as vitamin E acetate, and saponins.

The vaccine according to the invention comprises an effective dosage of the classical IBDV mutant as the active component, i.e. an amount of immunising IBDV material that will induce immunity in the vaccinated birds against challenge by a virulent virus. Immunity is defined herein as the induction of a significant higher level of protection in a population of birds after vaccination compared to an unvaccinated group.

Typically, the live vaccine according to the invention can be administered in a dose of 10⁰-10⁹ TCID₅₀ per animal, preferably in a dose ranging from 10³-10⁶ TCID₅₀ per animal. Inactivated vaccines may contain the antigenic equivalent of 10⁶-10¹⁰ TCID₅₀ per animal.

Inactivated vaccines are usually administered parenterally, e.g. intramuscularly or subcutaneously.

Although, the IBDV vaccine according to the present invention may be used effectively in chickens, also other poultry such as turkeys, guinea fowl and partridges may be successfully vaccinated with the vaccine. Chickens include broilers, pullets, reproduction stock and laying stock.

The age of the animals receiving a live or inactivated vaccine according to the invention is the same as that of the animals receiving the conventional live- or inactivated IBDV vaccines. For example, broilers (free of maternally derived antibodies-MDA) may be vaccinated at one-day-old or in ovo, whereas broilers with high levels of MDA are preferably vaccinated at 2-3 weeks of age. Laying stock or reproduction stock with low levels of MDA may be vaccinated at 1-10. days of age followed by booster vaccinations with inactivated vaccine on 6-12 and 16-20 weeks of age.

The invention also includes combination vaccines comprising, in addition to the classical IBDV mutant described above, one or more vaccine components of other pathogens infectious to poultry.

Preferably, the combination vaccine additionally comprises one or more vaccine strains of Mareks Disease virus (MDV), infectious bronchitis virus (IBV), Newcastle disease virus (NDV), egg drop syndrome (EDS) virus, turkey rhinotracheitis virus (TRTV) or reovirus.

### EXAMPLES

### Example 1

### Preparation of classic IBDV mutants and determination of monoclonal antibody reactivity

### Material and Methods

### Generation of mutated segment A

For site directed mutagenesis experiments pD78A (Mundt and Vakharia, supra, 1996; EP application 887412) was used. To this end pD78A was EcoRl/Kpnl cleaved and the segment A containing fragment was ligated into appropriately cleaved pBlueScript KS+ to obtain pSK+-D78A. After preparation of single stranded DNA site directed experiments were performed according to Kunkel et al. (supra, 1987) using oligonucleotides as specified in Table 3. Oligonucleotides Mut 1, Mut 2, Mut3, Mut4, Mut5, Mut6, Mut7, Mut8, Mut9, Mut10, Mut11 were used to generate mutated plasmids pMut1, pMut2, pMut3, pMut4, pMut5, pMut6, pMut7, pMut8, pMut9, pMut10, and pMut11, respectively. Using this eleven mutated plasmids single stranded DNA was prepared and used together with single stranded DNA of pSK+-D78A in site directed mutagenisis experiments . These experiments were performed with one (P222S or R339S) or two oligonucleotides (P222S and R339S) in one experiment to obtain one or two exchanges of base triplets. Obtained mutagenised plasmids (shown in Table 4) were sequenced and used for further experiments.

### Transfection of cRNA, immunoflourescence assays and passaging of generated virus

For *in vitro* transcription plasmids containing pD78A and mutagenised plasmids were linearized by cleavage with *Bsr*G 1. pP2B (Mundt & Vakharia, supra, 1996; EP application 887412) was linearized using Pst I. Further treatment of linearized DNA, transcription and transfection of RNA into BHK 21 cells were carried out as described by Mundt (J. Gen. Virol. 80, 2067-2076, 1999). For immunoflourescence assay BHK21 cells grown in 24 well tissue culture plates were transfected and 24h after transfection aceton/ methanol (50%/50%) fixed for 5 min and dried. Fixed cells were incubated with monoclonal antibodies 67, B69, 57, R63 and rabbit anti-IBDV serum (Mundt et al., J. Gen. Virol. 76, 437-443, 1995), respectively, diluted in phosphate buffered saline (PBS) for 30 min and rinsed trice with PBS. Cells were now incubated for 30min in PBS diluted DTAF-conjugated goat anti-rabbit IgG or DTAF-conjugated goat anti-mouse IgG (Dianova, Hamburg, Germany) followed by three washes using PBS and one wash with destilled water. After air drying cells were mounted in 2.5% 1.4.-Diazobicyclo(2.2.2.)-octane (DABCO, Sigma, Deisenhofen, Germany) containing PBS with 90% glycerol. Flourescence was visualised using a immerse flourescence microscope.

For passaging of generated virus BHK21 cells grown in 6 well tissue culture plates were transfected in parallel to the transfection experiments in 24 well tissue culture plates and incubated for 24h - 48h. After freeze/thaw at -70° for at least one hour obtained supernatant was centrifuged at 6400 x g for 10min and passaged onto QM cells grown in 25cm² tissue culture flask until CPE was visible. Supernatant was obtained as described above, aliquoted and stored at -70°C. For analysis of viability and presence of reactivity with the mmAb QM-cells grown in 24 well tissue culture plates were infected with one aliqout and incubated for 24h. Immunoflourescence assay was performed as described above.

### Growth analysis of generated virus in cell culture

To monitor growth CEC grown in 24 well tissue culture plate were infected with selected IBDV at a MOl of 1 for 1 h at 37°C. Thereafter, inoculum was removed , cells were washed with medium and 1ml medium was added. Supernatants were harvested separately immediately thereafter (0 h), and after 8, 12, 16, 24 and 36 h of incubation at 37°C and stored at -70°C. Virus titers were obtained by determination of TClD50 using QM-cells (Quail muscle cells) grown in 96 well tissue culture plates. To this end supernatants were thawed and titrated in log10 steps. 100µl each of the appropriate dilution was pipetted into four wells of a tissue culture plate followed by addition of 100µl QM cells suspension (10⁶cells/ml). The plates were incubated at 37°C. After five days wells with CPE were couted as positive and TCID50 was determined following Spaerman (Brit. J. Psychol., 2, 227-242, 1908) and Karber (Arch. Exp. Path. Pharmak.., 162, 480-487, 1931). Average values and standard deviations of three independent experiments were calculated.

### Results

### Influence of the exchange of amino acids in the variable region of VP2 on the reactivity of monoclonal antibodies

Amino acids located in the sequence of strain D78 at position 222 (proline), 318 (glycine), 321 (alanine), and 323 (aspartate) were exchanged to amino acids serine, threonine

(P222S, P222T), aspartate, asparagine (G318D, G318N, glutamate (A321E), and glutamate (D323E), respectively, in different combinations (see Table 4). Exchange of proline in position 222 to serine resulted in an additional reactivity of moab 67 if the aa sequence of aa from position 318 to 323 was of following combinations: GGQAGD, DGQAGD, DGQAGE, GGQAGE, NGQAGE. The remaining combinations of the aa sequence from position 318 to 323 (DGQEGD, DGQEGE, GGQEGD, GGQEGE, NGQAGD, NGQEGD, NGQEGE) seems to prevent the folding of the epitope characterized by moab 67 even if proline at position 222 was exchanged to serine. Binding of moab 57 was detected after exchange of aa 321 from alanine to glutamate independent if amino acid 222 (proline), 318 (glycine), and 323 (aspartate). But the exchange of arginine to serine at position 330 influenced the presence of the 57 epitope. Here if the performed exchange (R330S) was performed in presence of the combinations DGQEGD, NGQEGD, and NGQEGE, respectively, no reactivity with moab 57 was detected after co-tramsfection experiments. In contrast, the exchange R330S showed no influence on the reactivity with moab 57 in presence of the combination GGQEGD. The presence of reactivity of moab 57 and R63 excluded each other in the performed experiments since if the 57 epitope was present the R63 epitope was absent. Furthermore reactivity with moab R63 after co-transfection experiments was recorded after usage of plasmids encoding combinations GGQAGD, DGQAGD, DGQAGE, GGQAGE, NGQAGD, and NGQAGE from aa 318 to 323 located in the VP2 region independent if aa 222 (proline) or aa 330 (argenine) was exchanged. Translated protein of cRNA of plasmids encoding the amino acid sequence DGQEGE or GGQEGE from position 318 to 323 of the polyprotein gene reacted only with the moab 69. Here also the exchange of aa 222 and/or 330 seems to have no influence on the reactivity. After all transfection experiments cells were freezed/thawed and the obtained supernatant was passaged. In each case viable virus was generated indicating that the performed mutagenised amino acids had no influence on viability and infectivity of cell culture of the virus.

### Analysis of growth in cell culture

To test if amino acid exchange influence the growth of mutated virus several mutated IBDV (D78, Mut1, Mut2, PS-D78, PS-Mut1, PS-Mut2, Mut 10, Mut11) were analysed. To this end generated IBDV were selected which contain the same reactivity pattern with the used panel of moab. As shown in Figure growth of virus was influenced by exchange of amino acids in certain regions. Exchange of amino acid 222 from proline to serine showed no influence in growth in cell culture. In contrast, exchange in the region from amino acid 318 to 323 influenced the growth of the investigated mutants. These mutants growth to lower titers at all time points investigated indicating that the region from aa 318 to aa323 is of importance for growth in cell culture.

**Table 3: Oligonucleotides used in site-directed mutagenesis**

| Sequence | Orientation | Position | Amino acid exchange | Name | SEQ ID No. |
|---|---|---|---|---|---|
| 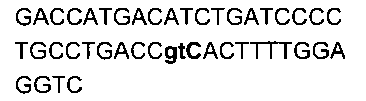 | antisense | 1069-1113 | G318D | Mut1 | 20 |
| 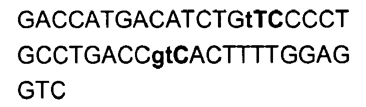 | antisense | 1069-1113 | G318D D323E | Mut2 | 21 |
| 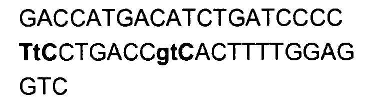 | antisense | 1069-1113 | G318D A321E | Mut3 | 22 |
| 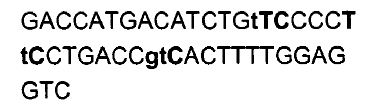 | antisense | 1069-1113 | G318D A321E D323E | Mut4 | 23 |
| 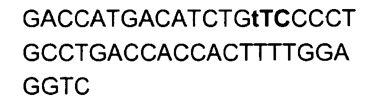 | antisense | 1069-1113 | D323E | Mut5 | 24 |
| 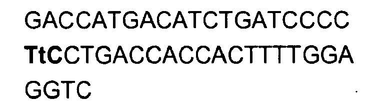 | antisense | 1069-1113 | A321E | Mut6 | 25 |
| 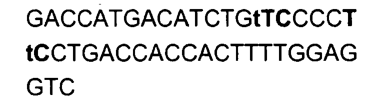 | antisense | 1069-1113 | A321E D323E | Mut7 | 26 |
| 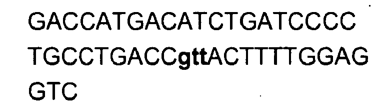 | antisense | 1069-1113 | G318N | Mut8 | 27 |
| 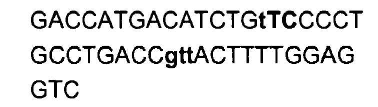 | antisense | 1069-1113 | G318N D323E | Mut9 | 28 |
| 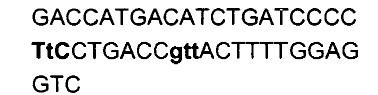 | antisense | 1069-1113 | G318N A321E | Mut10 | 29 |
| 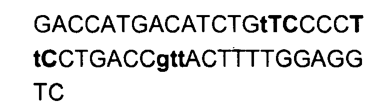 | antisense | 1069-1113 | G318N A321E D323E | Mut11 | 30 |
| 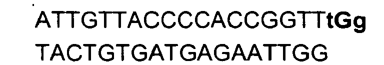 | antisense | 772-810 | P222T | P222T | 31 |
| 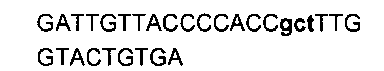 | antisense | 782-811 | P222S | P222S | 32 |
| 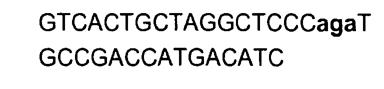 | antisense | 1102-1137 | R330S | R330S | 33 |

**Table 4: Results of site-directed mutagenesis, transfection experiments and immunofluorescence assay**

| Oligo nucleotides^{a} | Plasmids^{b} | aa-sequence^{c} | 57 | R63 | 67 | B69 | Viable | SEQ ID No. |
|---|---|---|---|---|---|---|---|---|
| | pD78A | GGQAGD | - | + | - | + | + | 1 |
| P222S | pD78A-P222S | GGQAGD | - | + | + | + | + | 1 |
| P222T | pD78A-222T | GGQAGD | | | | | | 1 |
| R330S | pD78A-R330S | GGQAGD | - | + | - | + | + | 1 |
| P222S, R330S | pD78A-P222S - R330S | GGQAGD | - | + | + | + | + | 1 |
| Mut1 | pMut1 | **D**GQAGD | - | + | - | + | + | 3 |
| Mut1, P222S | pMut1-P222S | **D**GQAGD | - | + | + | + | + | 3 |
| Mut1, R330S | pMut1-R330S | **D**GQAGD | - | + | - | + | + | 3 |
| Mut1, P222S, R330S | pMut1- P222S - R330S | **D**GQAGD | - | + | + | + | + | 3 |
| Mut2 | pMut2 | **D**GQAG**E** | - | + | - | + | + | 4 |
| Mut2, P222S | pMut2-P222S | **D**GQAG**E** | - | + | + | + | + | 4 |
| Mut2, P222T | pMut2-P222T | **D**GQAG**E** | | | | | | 4 |
| Mut2, R330S | pMut2-R330S | **D**GQAG**E**- | - | + | - | + | + | 4 |
| Mut2, P222S, R330S | pMut2- P222S - R330S | **D**GQAG**E** | - | + | + | + | + | 4 |
| Mut3 | pMut3 | **D**GQ**E**GD | + | - | - | + | + | 7 |
| Mut3, P222S | pMut3-P222S | **D**GQ**E**GD | + | - | - | + | + | 7 |
| Mut3, R330S | pMut3-R330S | **D**GQ**E**GD | - | + | - | + | + | 7 |
| Mut4 | pMut4 | **D**GQEG**E** | - | - | - | + | + | 34 |
| Mut4, P222S | pMut4-P222S | **D**GQEG**E** | - | - | - | + | + | 34 |
| Mut4, R330S | pMut4-R330S | **D**GQEG**E** | - | - | - | + | + | 34 |
| Mut5 | pMut5 | GGQAG**E** | - | + | - | + | + | 2 |
| Mut5, P222S | pMut5-P222S | GGQAG**E** | - | + | + | + | + | 2 |
| Mut5, P222T | pMut5-P222T | GGQAG**E** | | | | | | 2 |
| Mut5, R330S | pMut5-R330S | GGQAG**E** | - | + | - | + | + | 2 |
| Mut6 | pMut6 | GGQ**E**GD | + | - | - | + | + | 6 |
| Mut6, P222S | pMut6-P222S | GGQ**E**GD | + | - | - | + | + | 6 |
| Mut6, R330S | pMut6-R330S | GGQ**E**GD | + | - | - | + | + | 6 |
| Mut7 | pMut7 | GGQ**E**G**E** | - | - | - | + | + | 35 |
| Mut7, P222S | pMut7-P222S | GGQ**E**G**E** | - | + | - | + | + | 35 |
| Mut7, R330S | pMut7-R330S | GGQ**E**G**E** | - | - | - | + | + | 35 |
| Mut8 | pMut8 | **N**GQAGD | - | + | - | + | + | 15 |
| Mut8, P222S | pMut8-P222S | **N**GQAGD | - | + | - | + | + | 15 |
| Mut8, R330S | pMut8-R330S | **N**GQAGD | - | + | - | + | + | 15 |
| Mut9 | pMut9 | **N**GQAG**E** | - | + | - | + | + | 16 |
| Mut9, P222S | pMut9-P222S | **N**GQAG**E** | - | + | + | + | + | 16 |
| Mut9, P222T | pMut9-P222T | **N**GQAG**E** | | | | | | 16 |
| Mut9,R330S | pMut9-R330S | **N**GQAG**E** | - | + | - | + | + | 16 |
| Mut10 | pMut10 | **N**GQ**E**GD | + | - | - | + | + | 8 |
| Mut10, P222S | pMut10-222S | **N**GQ**E**GD | + | - | - | + | + | 8 |
| Mut10, R330S | pMut10-330S | **N**GQ**E**GD | - | + | - | + | + | 8 |
| Mut11 | pMut11 | **N**GQ**E**G**E** | + | - | - | + | + | 9 |
| Mut11, P222S | pMut11-222S | **N**GQ**E**G**E** | + | - | - | + | + | 9 |
| Mut11, R330S | pMut11-330S | **N**GQ**E**G**E** | - | + | - | + | + | 9 |

| | | | | | | | | |
|---|---|---|---|---|---|---|---|---|
| ^{a} Oligonucleotides used for site directed mutagenesis experiments ^{b} Obtained mutagenised plasmids used for transfection experiments ^{c} Amino acids sequence located at amino acid positions 318-323 is shown in single letter code and exchanged amino acids in comparison to the D78 sequence are bold typed | | | | | | | | |

### Example 2

### Antigenic properties of the classic IBDV mutants

### Material and Methods

### Characterization of mutant virus by neutralization assay

To test wether the generated virus was neutralized by monoclonal antibodies neutralization assay was performed essentially as described (Schröder et al., J. Gen. Virol., 81, 533-540, 2000). In brief, 100µl of a virus solution containing 750 TCID₅₀/100µl was pipetted into each well of a 96 well tissue culture plate with the exception of the first well in each row. Then, 100µl of either the different moabs (67, B69, 57, R63) or a polyclonal rabbit anti-IBDV serum was pipetted into the empty first well of each row. To the antibody containing wells 100µl of a virus suspension containing 1500 TClD₅₀/100µl was added then. After mixing virus and serum serial dilutions were made by serially transferring 100µl/well. After incubation for 1 hour at room temperature 100µl of QM cells (10⁶ cells/ml) were added to each well and incubated at 37°C. Six days later wells were scored for the presence of CPE. The end point of the VN-test for a serum sample was determined to be the reciprocal of the highest dilution, expressed in log2, in which no CPE was visible.

### Results

### Antigenic properties in neutralization asssay

To assay if generated mutants could be neutralized by the appropriate monoclonal antibodies neutralization assays were peformed. For this assay pairs of virus were selected which showed either a different mAb pattern based on one amino acid exchange (D78, PS-D78; Mut1, PS-Mut1; Mut2, PS-Mut2) or the same pattern but different amino acid sequences (D78, Mut1, Mut2,; PS-D78, PS-Mut1, PS-Mut2; Mut10, Mut11). The results showed (Table 5) that neutralization occurred in most cases (D78, Mut1, Mut2, PS-D78, PS-Mut1, PS-Mut2, Mut11) in the same pattern as in the fluorescence assay. One exception was Mut 11, which was not neutralized by mAb 57, although it was positive in fluorescence assay indicating that the epitope was present but lost his neutralising property.

**Table 5: Neutralization assay of IBDV mutants using polyclonal serum and monoclonal antibodies**

| Virus^{a} | Panel pattern in IIFA^{b} | | | | Antibodies used in neutralization assay^{c} | | | | |
|---|---|---|---|---|---|---|---|---|---|
| | 57 | R63 | 67 | B69 | 57 | R63 | 67 | B69 | anti -IBDV |
| rD78 | - | + | - | + | <2^{d} | >2¹² | <2 | 2¹² | 2¹² |
| PS-D78 | - | + | + | + | <2 | >2¹² | 2¹⁰ | 2¹¹ | 2¹² |
| Mut1 | - | + | - | + | <2 | >2¹² | <2 | 2¹⁰ | 2⁸ |
| PS-Mut1 | - | + | + | + | <2 | >2¹² | 2¹¹ | 2¹⁰ | 2¹⁰ |
| Mut2 | - | + | - | + | <2 | >2¹² | <2 | 2¹¹ | 2⁹ |
| PS-Mut2 | - | + | + | + | <2 | 2¹² | 2¹² | 2¹⁰ | 2⁷ |
| Mut10 | + | - | - | + | 2¹¹ | <2 | <2 | 2¹⁰ | 2⁹ |
| Mut11 | + | - | - | + | <2 | <2 | <2 | 2¹² | 2⁸ |

| | | | | | | | | | |
|---|---|---|---|---|---|---|---|---|---|
| ^{a} recombinant IBDV used in the neutralization assay ^{b} results of the panel pattern in the indirect immunflourescence assay using moabs (57, R63, 67, B69) as shown in table 4. ^{c} In the neutralization assay four moabs (57, R63, 67, B69) and one polyclonal rabbit anti -IBDV serum. ^{d} neutralization titer in log₂ of the diluted serum | | | | | | | | | |

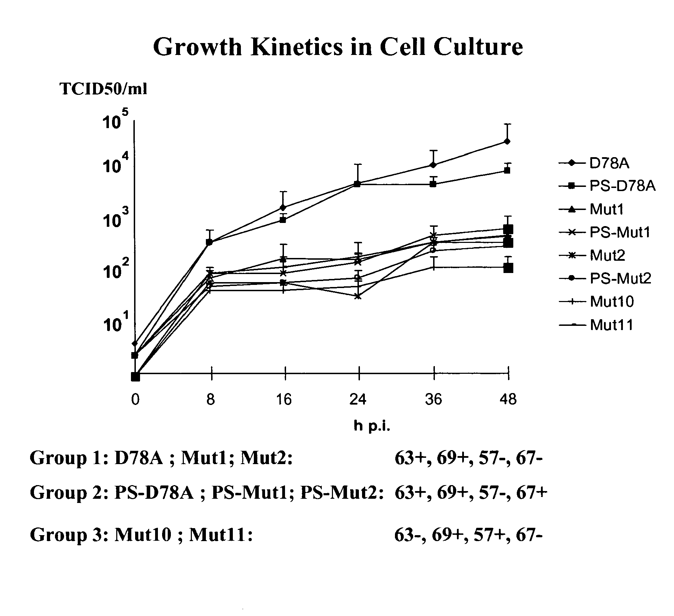

## Claims

1. A method for the preparation of a classic infectious bursal disease virus (IBDV) mutant that expresses a VP2 protein that binds with monoclonal antibody (moab) B69 and moab 67, secreted by hybridoma cell lines HB-9437, and HB-11122 respectively, deposited at the ATCC, Rockville, USA, **characterised in that** one or more mutations are introduced in a VP2 coding region of a classic IBDV strain, such that
(i) a codon for the amino acid at position 222 encodes serine or threonine, and
(ii) a nucleotide sequence encoding an amino acid sequence for positions 318 - 323 encodes an amino acid sequence shown in any of the SEQ ID No. 1 - 5.

2. A method according to claim 1, **characterised in that** the mutation is introduced in the codon for the amino acid at position 222 in a VP2 coding region of a classic IBDV strain that comprises a nucleotide sequence encoding the amino acid sequence shown in SEQ ID No. 1.

3. A method according to claim 1 or 2, **characterised in that** the VP2 protein also binds with moab R63, secreted by hybridoma cell line HB-9490, deposited at the ATCC, Rockville, USA.

4. A method according to claims 1 - 3, **characterised in that** the VP2 coding region comprises a codon for the amino acid at position 330 encoding arginine or serine.

5. A method according to claims 1 - 4, **characterised in that** the one or more mutations are introduced in a VP2 coding region of IBDV strain D78.

6. A method according to claims 1 - 5, **characterised in that** the one or more mutations are introduced in a genomic segment A of a classic IBDV, preferably of IBDV strain D78.

7. A classic IBDV mutant obtainable by the method according to any one of the claims 1 - 6, **characterised in that** one or more mutations are introduced in the VP2 coding region of a classic IBDV strain, such that
(i) a codon for the amino acid at position 222 encodes threonine, and
(ii) a nucleotide sequence encoding an amino acid sequence for positions 318 - 323 encodes an amino acid sequence shown in any of the SEQ ID No. 1-5.

8. A classic IBDV mutant obtainable by the method according to any one of the claims 1 or 3 - 6, **characterised in that** one or more mutations are introduced in the VP2 coding region of a classic IBDV strain, such that
(i) a codon for the amino acid at position 222 encodes serine or threonine, and
(ii) a nucleotide sequence encoding an amino acid sequence for positions 318 - 323 encodes an amino acid sequence shown in any of the SEQ ID No. 2-5.

9. A classic IBDV mutant according to claims 7 or 8, **characterised in that** the VP2 protein also binds with moab R63, secreted by hybridoma cell line HB-9490, deposited at the ATCC, Rockville, USA.

10. A classic IBDV mutant according to claims 7 - 9, **characterised in that** the coding region comprises a codon for the amino acid at position 330 encoding arginine or serine.

11. A classic IBDV mutant according to claims 7 - 10, **characterised in that** the mutant comprises one or more mutations in a VP2 coding region of IBDV strain D78.

12. A classic IBDV mutant according to claims 7 - 11, **characterised in that** the mutant comprises a genomic segment A of a classic IBDV, preferably of IBDV strain D78.

13. A vaccine for use in the protection of poultry against disease caused by IBDV infection, **characterised in that** the vaccine comprises a classic IBDV mutant according to claims 7 - 12, together with a pharmaceutically acceptable carrier or diluent.

14. A vaccine according to claim 13, **characterised in** the classic IBDV mutant is in a live form.

15. A vaccine according to claims 13 or 14, **characterised in that** the vaccine further comprises one or more vaccine components of other pathogens infectious to poultry.

16. A vaccine according to claims 13 - 15, **characterised in that** the vaccine comprises an adjuvant.

17. A method for the preparation of a vaccine for use in the protection of poultry against disease caused by IBDV infection, **characterised in that** a classic IBDV mutant according to claims 7 - 12 is mixed with a pharmaceutically acceptable carrier or a diluent.

## Patentansprüche

1. Verfahren zur Herstellung einer Mutante eines klassischen Infektiösen-Bursitis-Virus (IBDV), die ein VP2-Protein exprimiert, das an die monoklonalen Antikörper moab B69 und moab 67, die von den Hybridomzellinien HB-9437 bzw. HB-11122, welche bei der ATCC, Rockville, USA, hinterlegt sind, sezerniert werden, bindet, **dadurch gekennzeichnet, daß** eine oder mehrere Mutationen in eine VP2-Codierregion eines klassischen IBDV-Stamms eingeführt werden, so daß
(i) ein Codon für die Aminosäure in Position 222 für Serin oder Threonin codiert und
(ii) eine Nukleotidsequenz, die für eine Aminosäuresequenz für die Positionen 318 - 323 codiert, für eine Aminosäuresequenz gemäß einer der SEQ ID No. 1 - 5 codiert.

2. Verfahren nach Anspruch 1, **dadurch gekennzeichnet, daß** die Mutation in das Codon für die Aminosäure in Position 222 in einer VP2-Codierregion eines klassischen IBDV-Stamms, die eine Nukleotidsequenz, die für die Aminosäuresequenz gemäß SEQ ID No. 1 codiert, umfaßt, eingeführt wird.

3. Verfahren nach Anspruch 1 oder 2, **dadurch gekennzeichnet, daß** das VP2-Protein auch an moab R63, der von der Hybridomzellinie HB-9490, die bei der ATCC, Rockville, USA, hinterlegt ist, sezerniert wird, bindet.

4. Verfahren nach den Ansprüchen 1 - 3, **dadurch gekennzeichnet, daß** die VP2-Codierregion ein Codon für die Aminosäure in Position 330, die für Arginin oder Serin codiert, umfaßt.

5. Verfahren nach den Ansprüchen 1 - 4, **dadurch gekennzeichnet, daß** eine oder mehrere Mutationen in eine VP2-Codierregion des IBDV-Stamms D78 eingeführt werden.

6. Verfahren nach den Ansprüchen 1 - 5, **dadurch gekennzeichnet, daß** eine oder mehrere Mutationen in einen Genomabschnitt A eines klassischen IBDV, vorzugsweise IBDV-Stamm D78, eingeführt werden.

7. Klassische IBDV-Mutante, die nach dem Verfahren nach einem der Ansprüche 1 - 6 erhältlich ist, **dadurch gekennzeichnet, daß** eine oder mehrere Mutationen in die VP2-Codierregion eines klassischen IBDV-Stamms eingeführt werden, so daß
(i) ein Codon für die Aminosäure in Position 222 für Threonin codiert und
(ii) eine Nukleotidsequenz, die für eine Aminosäuresequenz für die Positionen 318 - 323 codiert, für eine Aminosäuresequenz gemäß einer der SEQ ID No. 1 - 5 codiert.

8. Klassische IBDV-Mutante, die nach dem Verfahren nach einem der Ansprüche 1 oder 3 - 6 erhältlich ist, **dadurch gekennzeichnet, daß** eine oder mehrere Mutationen in die VP2-Codierregion eines klassischen IBDV-Stamms eingeführt werden, so daß
(i) ein Codon für die Aminosäure in Position 222 für Serin oder Threonin codiert und
(ii) eine Nukleotidsequenz, die für eine Aminosäuresequenz für die Positionen 318 - 323 codiert, für eine Aminosäuresequenz gemäß einer der SEQ ID No. 2 - 5 codiert.

9. Klassische IBDV-Mutante nach Anspruch 7 oder 8, **dadurch gekennzeichnet, daß** das VP2-Protein auch an moab R63, der von der Hybridomzellinie HB-9490, die bei der ATCC, Rockville, USA, hinterlegt ist, sezerniert wird, bindet.

10. Klassische IBDV-Mutante nach den Ansprüchen 7 - 9, **dadurch gekennzeichnet, daß** die Codierregion ein Codon für die Aminosäure in Position 330, die für Arginin oder Serin codiert, umfaßt.

11. Klassische IBDV-Mutante nach den Ansprüchen 7 - 10, **dadurch gekennzeichnet, daß** die Mutante eine oder mehrere Mutationen in einer VP2-Codierregion des IBDV-Stamms D78 umfaßt.

12. Klassische IBDV-Mutante nach den Ansprüchen 7 - 11, **dadurch gekennzeichnet, daß** die Mutante einen Genomabschnitt A eines klassischen IBDV, vorzugsweise IBDV-Stamm D78, umfaßt.

13. Impfstoff für die Verwendung im Schutz von Geflügel gegen durch IBDV-Infektion verursachte Krankheiten, **dadurch gekennzeichnet, daß** der Impfstoff eine klassische IBDV-Mutante nach den Ansprüchen 7 - 12 zusammen mit einem pharmazeutisch unbedenklichen Träger oder Streckmittel umfaßt.

14. Impfstoff nach Anspruch 13, **dadurch gekennzeichnet, daß** die klassische IBDV-Mutante in lebender Form vorliegt.

15. Impfstoff nach Anspruch 13 oder 14, **dadurch gekennzeichnet, daß** der Impfstoff weiterhin einen oder mehrere Impfstoffkomponenten von anderen für Geflügel infektiösen Pathogenen umfaßt.

16. Impfstoff nach den Ansprüchen 13 - 15, **dadurch gekennzeichnet, daß** der Impfstoff ein Adjuvans umfaßt.

17. Verfahren zur Herstellung eines Impfstoffs für die Verwendung im Schutz von Geflügel gegen durch IBDV-Infektion verursachte Krankheiten, **dadurch gekennzeichnet, daß** eine klassische IBDV-Mutante nach den Ansprüchen 7 - 12 mit einem pharmazeutisch unbedenklichen Träger oder Streckmittel vermischt wird.

## Revendications

1. Procédé pour la préparation d'un mutant du virus de la bursite infectieuse (IBDV) classique exprimant une protéine VP2 qui se lie à des anticorps monoclonaux (moab) B69 et moab 67 sécrétés par des lignées de cellules d'hybridome HB-9437 et HB-11122 respectivement, déposées auprès de l'ATCC, Rockville, USA, **caractérisé en ce qu'**une ou plusieurs mutation(s) est (sont) introduite(s) dans une région codante VP2 d'une souche d'IBDV classique, de sorte que :
(i) un codon pour l'acide aminé à la position 222 code pour une sérine ou une thréonine et
(ii) une séquence nucléotidique, codant pour une séquence d'acides aminés aux positions 318 à 323, code pour une séquence d'acides aminés présentée dans l'une quelconque des SEQ ID n° 1 à 5.

2. Procédé, selon la revendication 1, **caractérisé en ce que** la mutation est introduite dans le codon pour l'acide aminé en position 222 dans une région codante VP2 d'une souche d'IBDV classique, laquelle comprend une séquence nucléotidique codant pour la séquence d'acides aminés présentée à la SEQ ID n° 1.

3. Procédé, selon la revendication 1 ou la 2, **caractérisé en ce que** la protéine VP2 se lie également à un moab R63 sécrété par la lignée de cellules d'hybridome HB-9490, déposée auprès de l'ATCC, Rockville, USA.

4. Procédé, selon les revendications 1 à 3, **caractérisé en ce que** la région codante VP2 comprend un codon pour l'acide aminé en position 330 qui code pour une arginine ou une sérine.

5. Procédé, selon les revendications 1 à 4, **caractérisé en ce qu'**une ou plusieurs mutation(s) est (sont) introduite(s) dans une région codante VP2 d'une souche D78 d'IBDV.

6. Procédé, selon les revendications 1 à 5, **caractérisé en ce qu'**une ou plusieurs mutation(s) est (sont) introduite(s) dans un segment génomique A d'un IBDV classique, de préférence d'une souche D78 d'IBDV.

7. Mutant d'IBDV classique que l'on peut obtenir par le procédé selon l'une quelconque des revendications 1 à 6, **caractérisé en ce qu'**une ou plusieurs mutation(s) est (sont) introduite(s) dans la région codante VP2 d'une souche d'IBDV classique, de sorte que :
(i) un codon pour l'acide aminé à la position 222 code pour une thréonine et
(ii) une séquence nucléotidique, codant pour une séquence d'acides aminés aux positions 318 à 323, code pour une séquence d'acides aminés présentée dans l'une quelconque des SEQ ID n° 1 à 5.

8. Mutant d'IBDV classique que l'on peut obtenir par le procédé selon l'une quelconque des revendications 1 ou 3 à 6, **caractérisé en ce qu'**une ou plusieurs mutation(s) est (sont) introduite(s) dans la région codante VP2 d'une souche d'IBDV classique, de sorte que :
(i) un codon pour l'acide aminé à la position 222 code pour une sérine ou une thréonine et
(ii) une séquence nucléotidique, codant pour une séquence d'acides aminés aux positions 318 à 323, code pour une séquence d'acides aminés présentée dans l'une quelconque des SEQ ID n° 2 à 5.

9. Mutant d'IBDV classique, selon les revendications 7 ou 8, **caractérisé en ce que** la protéine VP2 se lie aussi à un moab R63 sécrété par la lignée de cellules d'hybridome HB-9490, déposée auprès de l'ATCC, Rockville, USA.

10. Mutant d'IBDV classique, selon les revendications 7 à 9, **caractérisé en ce que** la région codante comprend un codon pour l'acide aminé en position 330 qui code pour une arginine ou une sérine.

11. Mutant d'IBDV classique, selon les revendications 7 à 10, **caractérisé en ce que** le mutant comprend une ou plusieurs mutation(s) dans une région codante VP2 d'une souche D78 d'IBDV.

12. Mutant d'IBDV classique, selon les revendications 7 à 11, **caractérisé en ce que** le mutant comprend un segment génomique A d'un IBDV classique, de préférence d'une souche D78 d'IBDV.

13. Vaccin destiné à être utilisé dans la protection des volailles contre une maladie provoquée par une infection à IBDV, **caractérisé en ce que** le vaccin comprend un mutant d'IBDV classique, selon les revendications 7 à 12, conjointement avec un véhicule ou un diluant acceptable d'un point de vue pharmaceutique.

14. Vaccin, selon la revendication 13, **caractérisé en ce que** le mutant d'IBDV classique se trouve sous une forme vivante.

15. Vaccin selon les revendications 13 ou 14, **caractérisées en ce que** le vaccin comprend en outre un ou plusieurs composant(s) de vaccin provenant d'autres pathogènes infectieux pour les volailles.

16. Vaccin selon les revendications 13 à 15, **caractérisées en ce que** le vaccin comprend un adjuvant.

17. Procédé pour la préparation d'un vaccin destiné à être utilisé dans la protection des volailles contre une maladie provoquée par une infection à IBDV, **caractérisé en ce qu'**un mutant d'IBDV classique, selon les revendications 7 à 12, est mélangé à un véhicule ou un diluant acceptable d'un point de vue pharmaceutique.
